# EUROPEAN PATENT APPLICATION

(11) **EP 2 072 057 A1**
(43) Date of publication of application: **24.06.2009**
(21) Application number: 07024962.8
(22) Date of filing: 21.12.2007
(51) Int. Cl.: A61K 38/48

(54) **Early administration of Botulinum toxin in the treatment of cerebrovascular event and spinal cord injury**

(71) Applicant: Merz Pharma GmbH & Co. KGaA, 60318 Frankfurt (DE)
(72) Inventor: Durand-Lagarde, Marianne,Dr., 60318 Frankfurt (DE); Jost, Wolfgang, Prof. Dr., 65191 Wiesbaden (DE)
(74) Representative: Ricker, Mathias

(57) **Abstract**

The present invention relates to a *Clostridium botulinum* neurotoxin for the treatment of spasticity related to a cerebrovascular event or to a cerebral or spinal cord injury, wherein onset of treatment is less than 11 days after the cerebrovascular event or cerebral or spinal cord injury. Moreover, the present invention relates to a method of treatment of spasticity related to a cerebrovascular event or spinal cord injury, the method comprising administering to a patient a composition comprising an effective amount of a Clostridium botulinum toxin, wherein (a) the composition comprises: i. the neurotoxic component of the Clostridium botulinum toxin complex, being free of the complexing proteins of the botulinum toxin complex, or ii. the botulinum toxin complex; or iii. mixtures thereof and (b) the composition is administered less than 11 days after the cerebrovascular event; and (c) the composition is administered by injection.

## Description

The present invention relates to a *Clostridium botulinum* neurotoxin for the treatment of spasticity related to a cerebrovascular event or to a cerebral or spinal cord injury, wherein onset of treatment is less than 11 days after the cerebrovascular event or cerebral or spinal cord injury. Moreover, the present invention relates to a method of treatment of spasticity related to a cerebrovascular event or spinal cord injury, the method comprising administering to a patient a composition comprising an effective amount of a Clostridium botulinum toxin, wherein (a) the composition comprises: i. the neurotoxic component of the Clostridium botulinum toxin complex, being free of the complexing proteins of the botulinum toxin complex, or ii. the botulinum toxin complex; or iii. mixtures thereof and (b) the composition is administered less than 11 days after the cerebrovascular event; and (c) the composition is administered by injection.

Despite its toxic effects, botulinum toxin complex has been used as a therapeutic agent in a large number of diseases. Botulinum toxin serotype A was approved for human use in the United States in 1989 for the treatment of strabism, blepharospasm, and other disorders. It is commercially available as Botulinum toxin A protein complex, for example, under the tradename BOTOX^{®} (Allergan Inc) or under the tradename DYSPORT^{Ⓡ} (Ipsen Ltd). For therapeutic application e.g. the complex is injected directly into the muscle to be treated. At physiological pH, the neurotoxic component is thought to be released from the protein complex and the desired pharmacological effect takes place. The effect of Botulinum toxin is only temporary, which is the reason why repeated administration of Botulinum toxin may be required to maintain a therapeutic effect.

Acute cerebrovascular events like stroke are a leading cause for spasticity with 19% to 38% of patients are developing spasticity in upper and/or lower limbs after a stroke.

Spasticity is defined as a motor disorder characterized by a velocity-dependent increase in tonic stretch reflexes (muscle tone) with exaggerated tendon jerks, resulting from hyperexcitability of the stretch reflex as one component of the upper motoneuron syndrome. Treatment with botulinum toxin is becoming pharmacological treatment of first choice in focal spasticity. However, treatment of spasticity occurring after stroke usually starts months to years after the event when spasticity has been fully developed in the patients. The severity of spasticity is variable, i.e. it may increase or even decrease over time. Generally, however, severity appears to be stable after about 3 to 6 months following the stroke (*Formisano et al., 2005; Welmer et al., 2006*). When treated with appropriate rehabilitation measures, spasticity may be reduced over time. At present, however, no effective treatment exists, which would be suitable to prevent onset of the full symptoms of spasticity resulting from a cerebrovascular event or from a cerebral or spinal cord injury.

Thus, the technical problem underlying the present invention was to provide means and methods for preventing onset of the full symptoms of spasticity resulting from a cerebrovascular event or a cerebral or spinal cord injury.

The solution to this technical problem is achieved by providing the embodiments characterized in the claims.

Accordingly, the present invention relates to a *Clostridium botulinum* neurotoxin for the treatment of spasticity related to a cerebrovascular event or to a cerebral or spinal cord injury, wherein onset of treatment is less than 11 days after the cerebrovascular event or cerebral or spinal cord injury or the first onset of spasticity.

In a preferred embodiment of the present invention, treatment is initiated less than 11 days after onset of spasticity. Onset of spasticity as used herein refers to the observation of first signs of spasticity. First signs might be, for example, increased muscle tone.

Botulinum toxin is produced by the bacterium *Clostridium.* Botulinum toxins are released from lysed Clostridium cultures generally in the form of a sub-unit responsible for the toxic properties of the Botulinum toxin (the so-called "neurotoxic component") in association with other bacterial proteins, which together form a toxin complex - also designated "botulinum toxin complex". The botulinum toxin complex is metastable in nature, since its stability appears to depend on various factors such as e.g. salt concentration and/or pH. This complex usually contains additional, so-called "non-toxic" proteins, which we will refer to as "complexing proteins" or "bacterial proteins". The molecular weight of this complex may vary from about 300,000 to about 900,000 Da, i.e. from 300 kDa to about 900 kDa. The complexing proteins are, for example, various hemagglutinins. The proteins of this toxin complex are not toxic themselves but are believed to provide stability to the neurotoxic component and are responsible for oral toxicity in Botulinum intoxications. There are seven antigenically distinct serotypes of Botulinum toxin, namely Botulinum toxin A, B, C, D, E, F and G. Wherever the Botulinum toxin serotype A, B, C, D, E, F or G are mentioned, also known variants of the serotypes are encompassed, like serotypes A1, A2, A2, B1, B2, B3, C1, C2, C3, D1. D2, D3, E1, E2, E3, F1, F2, F3, or G1, G2, G3. As indicated in the introductory part of the application, there exist two commercially available formulation on the basis of the Botulinum toxin A protein complex, namely under the tradename BOTOX^{®} (Allergan Inc) and under the tradename DYSPORT^{®} (Ipsen Ltd)

The term "Botulinum toxin" as used throughout the present application, refer to the neurotoxic component devoid of any other Clostridial proteins, but also to the "Botulinum toxin complex". The term "Botulinum toxin" is used herein in cases when no discrimination between the toxin complex and the neurotoxic component is necessary or desired. "BoNT" or "NT" are common used abbreviations.

The "neurotoxic component" of the Botulinum toxin complex is initially formed as a single polypeptide chain, having in the case of serotype A a molecular weight of approximately 150 kDa. In other serotypes the neurotoxic component has been observed to vary between about 145 and about 170 kDa, depending on the bacterial source. In the case of serotype A, for example, proteolytic processing of the polypeptide results in an activated polypeptide in the form of a dichain polypeptide consisting of a heavy chain and a light chain, which are linked by a disulfide bond. In humans, the heavy chain mediates binding to pre-synaptic cholinergic nerve terminals and internalization of the toxin into the cell. The term "neurotoxic component" also includes functional homologs found in the other serotypes of Clostridium botulinum. In a preferred embodiment of the present invention, the neurotoxic component is devoid of any other *C. botulinum* protein, preferably also devoid of RNA, which might potentially be associated with the neurotoxic component.

The neurotoxic component may be the single chain precursor protein of approximately 150kDa or the proteolytically processed neurotoxic component, comprising the light chain (L_{c}) of approximately 50kDa and the heavy chain (H_{c}) of approximately 100kDa, which may be linked by one or more disulfide bonds (for a review see e.g. Simpson LL, Ann Rev Pharmacol Toxicol. 2004; 44:167-93). In humans, the heavy chain mediates binding to pre-synaptic cholinergic nerve terminals and internalization of the toxin into the cell. The light chain is believed to be responsible for the toxic effects, acting as zinc-endopeptidase and cleaving specific proteins responsible for membrane fusion (SNARE complex) (see e.g. Montecucco C., Shiavo G., Rosetto O: The mechanism of action of tetanus and Botulinum neurotoxins. Arch Toxicol. 1996; 18 (Suppl.): 342-354*)*).

The neurotoxic subunit of the Botulinum toxin complex is referred in this document as the "neurotoxic component" or the "neurotoxic component free of complexing proteins". A pharmaceutical composition comprising the neurotoxic component of Botulinum toxin type A in isolated form is commercially available in Germany from Merz Pharmaceuticals GmbH under the trademark Xeomin^{®}. The production of the neurotoxic component of Botulinum toxin type A and B are described, for example, in the international patent application WO 00/74703.

With regard to the composition and dosing of the medicament on the basis of Botulinum toxin, and in regard to the composition, dosing and frequency of administration of the medicament on the basis of the neurotoxic component of Botulinum toxin, reference is made to PCT/EP2007/005754.

In an preferred embodiment the Botulinum toxin is Botulinum toxin A. Serotype A is particularly preferred in the context of the present invention, because the duration of its therapeutic effect is superior when compared to the other serotypes. In the an even more preferred embodiment said Botulinum toxin is free of any complexing proteins (neurotoxic component), even more preferably it is the pure neurotoxic component serotype A. In addition thereto, modified as well as recombinant produced neurotoxic components of Botulinum toxins including the respective mutations, deletions, etc. are also within the scope of the present invention. With respect to suitable mutants, reference is made to WO 2006/027207 A1 and WO 2006/114308 A1, which are fully incorporated by reference herein. Furthermore, within the present invention, mixtures of various serotypes (in the form the neurotoxic component or recombinant form or both forms thereof, e.g. mixtures of Botulinum neurotoxins of types A and B) may be used. The present invention, however, also refers to neurotoxins which are chemically modified, e.g. by pegylation, glycosylation, sulfatation, phosphorylation or any other modification, in particular of one or more surface or solvent exposed amino acid(s).

In accordance with the teaching of the present invention it is particularly preferred that the medicament contains no proteins found in the botulinum toxin complex other than the neurotoxic component. The precursor of the neurotoxic component may be cleaved or uncleaved, however, it is preferred that the precursor has been cleaved into the heavy and the light chain. As pointed out elsewhere herein, the polypeptides may be of wild-type sequence or may be modified at one or more residues. Modification comprises chemical modification e.g. by glycosylation, acetylation, acylation or the like, which may be beneficial e.g. to the uptake or stability of the polypeptide. The polypeptide chain of the neurotoxic component may, however, alternatively or additionally be modified by addition, substitution or deletion of one or more amino acid residues.

The botulinum toxin, preferably the neurotoxic component referred to herein, may be part of a composition. This composition may contain the botulinum toxin/neurotoxic component as the sole active component or may contain additional pharmaceutically active components.

In a preferred embodiment of the present invention, the medicament comprises a serum albumin. In one embodiment, said serum albumin is a mammalian serum albumin, however, also comprised by this embodiment are albumin obtainable from other mammalians such as serum albumin derived from pig, bovine or primates as well as recombinantly produced albumins, as e.g. disclosed in EP 1 398 038. The albumin may be obtained by purification from the animal or by recombinant expression in a eukaryotic or prokaryotic cell. Alternatively, the albumin may be obtained by chemical synthesis. The aforementioned protein may be of wild-type sequence or may be modified at one or more residues. Modification comprises chemical modification e.g. by glycosylation, acetylation, acylation or the like, which may be beneficial e.g. to the uptake or stability of the polypeptide. The polypeptide chain of the albumin may, however, alternatively or additionally be modified by addition, substitution or deletion of one or more amino acid residues.

Preferably, said composition comprises the neurotoxic component of Botulinum toxin type A. Said composition is a reconstituted solution of the neurotoxic component of Botulinum toxin. Preferably, the composition further comprises sucrose or human serum albumin or both, still more preferably the ratio of human serum albumin to sucrose is about 1:5. In one embodiment, the composition is Xeomin^{®}. More preferably, said human serum albumin is recombinant human serum albumin. Alternatively, said composition is free of mammalian derived proteins such as human serum albumin. Any such solution may provide sufficient neurotoxin stability by replacing serum albumin with other non-proteinaceous stabilizers (infra).

The composition or pharmaceutical composition may contain additional pharmaceutically active components. "Pharmaceutical composition" is a formulation in which an active ingredient for use as a medicament or diagnostic is contained or comprised. Such pharmaceutical composition may be suitable for diagnostic or therapeutic administration (i.e. by intramuscular or subcutaneous injection) to a human patient. The pharmaceutical composition may be lyophilized or vacuum dried, reconstituted, or in solution. When reconstituted it is preferred that the reconstituted solution is prepared adding sterile physiological saline (0.9% NaCl).

The composition may comprise additional components such as a pH buffer, excipient, cryoprotectant, preservatives, stabilizer or any combination thereof.

The term "pH buffer" refers to a chemical substance being capable to adjust the pH value of a composition, solution and the like to a certain value or to a certain pH range. In one embodiment this pH range can be between pH 5 to pH 8, more preferably pH 7 to pH 8, even more preferably 7,2 to 7,6, and most preferably a pH of 7,4. The pH ranges given mentioned above are only typical examples and the actual pH may include any interval between the numerical values given above. Suitable buffers which are in accordance with the teaching of the present invention are e.g. sodium-phosphate buffer, sodium-acetate buffer, TRIS buffer or any buffer, which is suitable to buffer within the above pH-ranges.

The term "excipient" in this document refers to a substance present in a pharmaceutical composition other than the active pharmaceutical ingredient present in the pharmaceutical composition. An excipient can be a buffer, carrier, antiadherent, binder, disintegrant, filler, diluent, preservative, vehicle, cyclodextrin and/or bulking agent, such as albumin, gelatin, collagen and/or sodium chloride.

"Cryoprotectant" refers to excipients which result in the neurotoxic component in a reconstituted or aqueous solution pharmaceutical composition has greater than about 20%, 30%, 40%, 50%, 60%, 70%, 80%, 90%, and up to about 100% of the toxicity that the biologically active neurotoxic component had prior to being freeze-dried and reconstituted in the pharmaceutical composition.

The term "lyophilization" is used in this document for a treatment of a solution containing the neurotoxic component of the Botulinum toxin, whereas this solution is frozen and dried until only the solid components of the composition are left over. The freeze-dried product of this treatment is therefore defined in this document as "lyophilisate".

In this document the term "reconstitution" is defined as the process of solubilization of said freeze-dried composition of the neurotoxic component. This can be done by adding the appropriate amount of sterile water, e.g. if all necessary components are already contained in the lyophilisate. Or, if this is not the case, it can be done e.g. by adding a sterile saline-solution alone or if applicable with the addition of components comprising e.g. a pH buffer, excipient, cryoprotectant, preservative, analgesic stabilizer or any combination thereof. The saline of before mentioned "saline-solution" is a salt-solution, more preferably being a sodium-chloride (NaCl) solution, still more preferably being an isotonic sodium-chloride solution (i.e. a sodium-chloride concentration of 0,9%). The solubilization is carried out in such a manner that the final "reconstitution" is directly or indirectly, i.e. for example after dilution, administrable to the patient. Preferably, the neurotoxin is reconstituted in isotonic media. More preferably in isotonic saline. More preferably, said saline is sterile saline.

The terms "preservative" and "preservatives" refer to a substance or a group of substances, respectively, which prevent the growth or survival of microorganisms, insects, bacteria or other contaminating organisms within said composition. Preservatives also prevent said composition from undesired chemical changes. Preservatives which can be used in the scope of this patent are all preservatives of the state of the art known to the skilled person. Examples of preservatives that might be used include, inter alia, e.g. benzylic alcohol, benzoic acid, benzalkonium chloride, calcium propionate, sodium nitrate, sodium nitrite, sulphites (sulfur dioxide, sodium bisulfite, potassium hydrogen sulfite, etc.), disodium EDTA, formaldehyde, glutaraldehyde, diatomaceous earth, ethanol, methyl chloroisothiazolinone, butylated hydroxyanisole and/or butylated hydroxytoluene.

"Stabilizing", stabilizes" or "stabilization" means that the active ingredient, i.e., the neurotoxic component in a reconstituted or aqueous solution pharmaceutical composition has greater than about 20%, 30%, 40%, 50%, 60%, 70%, 80%, 90%, and up to about 100% of the toxicity that the biologically active neurotoxic component had prior to being incorporated into the pharmaceutical composition. The activity of the preparation may be determined as described elsewhere herein.

Examples of such stabilizers are gelatin or albumin, preferably of human origin or obtained from a recombinant source. Preferably, the albumin is in a concentration of from about 0.1 to about 10mg per 100 Units botulinum toxin, more preferably the albumin concentration is about 1mg per 100 Units botulinum toxin, wherein the term "about" is to be interpreted as ± 20%. Also preferred are albumin concentrations in the range of 0.1 to about 10mg per 800pg neurotoxic component, wherein an albumin concentration of about 1 mg per 800pg neurotoxic component is preferred. The term "about" is to be interpreted as ± 20% [w/w].

The stabilizers may be modified by chemical means or by recombinant genetics or both.

In a more preferred embodiment of the present invention, the stabilizer may be a nonproteinaceous stabilizing agent comprising hyaluronic acid or polyvinylpyrrolidone or polyethyleneglycol or a mixture of two or more thereof. Such composition is considered to be a safer composition possessing remarkable stability.

In a more preferred embodiment of the present invention, the pharmaceutical composition may comprise the neurotoxic component and a hyaluronic acid or a polyvinylpyrrolidone or a polyethleneglycol, such composition being optionally pH stabilized by a suitable pH buffer, in particular by a sodium acetate buffer, and / or a cryoprotectant polyalcohol.

Whether or not the pharmaceutical composition comprises, beside the neurotoxin component, additional components such as albumin, hyaluronic acid, a polyvinylpyrrolidone and/or a polyethyleneglycol stabilizer, the pharmaceutical composition retains its potency substantially unchanged for six month, one year, two year, three year and/or four year periods when stored at a temperature between about +8°C. and about -20°C. Additionally, the indicated pharmaceutical compositions may have a potency or percent recovery of between about 20% and about 100% upon reconstitution.

A pharmaceutical composition within the scope of the present invention may include the neurotoxic component one or more additional components. Preferably, the pharmaceutical compositions disclosed herein, has a pH of between about 4 and 7.5 when reconstituted or upon injection, more preferably between about pH 6.8 and pH 7.6 and most preferably between pH 7.4 and pH 7.6. Generally, the pharmaceutical composition of the present invention comprises neurotoxic component in a quantity of about 6pg to 30 ng. ***Alternative:** the pharmaceutical composition comprises neurotoxic component in a quantity of about 2 pg to 50 ng. Preferred quantity ranges are in the range of from 2 pg to 200 pg, 200 pg to 400 pg, 400 pg to 600 pg, 600 pg to 800 pg, 800 pg to 1 ng, 1 ng to 1,5ng, 1,5 ng to 2 ng, 2 ng to 2,5 ng, 2,5 ng to 3 ng, 3 to 3,5 ng, 3,5 to 4 ng, 4 ng to 4,5 ng, and 4, 5 to 5 ng.*

Preferably, the neurotoxic component has a biological activity of 50 to 250 LD50 units per ng neurotoxic component, as determined in a mouse LD50 assay. More preferably, the neurotoxic component has a biological activity of about 150 LD50 per ng neurotoxic component.

The pharmaceutical composition of the present invention may comprise a neurotoxin, and a hyaluronic acid. The hyaluronic acid stabilizes the neurotoxin. The pharmaceutical compositions disclosed herein may have a pH of between about 4 and 7.5 when reconstituted or upon injection. The hyaluronic acid in the instant pharmaceutical composition is preferably combined with the instant neurotoxic component in a quantity of 0.1 to 10 mg, especially 1 mg hyaluronic acid per ml in a 200 U/ml botulinum toxin solution.

More preferably, the subject solution also contains a 1-100 mM, especially 10 mM sodium acetate buffer.

In another preferred embodiment, the composition may contain a polyalcohol as cryoprotectant. Examples of polyalcohols that might be used include, e.g., inositol, mannitol and other non-reducing alcohols.

In particular those embodiments of the present invention's pharmaceutical composition not comprising a proteinaceous stabilizer, preferably do not contain trehalose or maltotriose or related sugar or polyhydroxy compounds which are sometimes used as cryoprotectants.

The polyvinylpyrrolidone in the instant pharmaceutical composition is preferably combined with the instant neurotoxic component in a quantity of 10 to 500 mg, especially 10 mg polyvinylpyrrolidone per ml in a 200 U/ml botulinum toxin solution. More preferably, the subject solution also contains a 1-100 mM, especially 10 mM sodium acetate buffer.

The polyethyleneglycol in the instant pharmaceutical composition is preferably combined with the instant neurotoxic component in a quantity of 10 to 500 mg, especially 100 mg polyethyleneglycol per ml in a 200 U/ml botulinum toxin solution. More preferably, the subject solution also contains a 1-100 mM, especially 10 mM sodium acetate buffer. This ratio of components is also applied in case lower concentrations of down to 25U/ml neurotoxic component solution.

Thus, the instant invention encompasses in a more preferred embodiment a neurotoxic component formulated in a pharmaceutical composition, which contains a hyaluronic acid stabilizer or a polyvinylpyrrolidone stabilizer or a polyethyleneglycol stabilizer or any combination thereof. Additionally, the pharmaceutical composition may contain a sodium acetate buffer system and/or an alcoholic cryoprotectant or both. In a further preferred embodiment the formulation is albumin free, and comprises as a stabilizer hyaluronic acid, polyvinylpyrrolidone (Kollidon^{®}), hydroxyethyl starch or alginate or a mixture of two or more of these. Said preferred composition comprises in addition to the mentioned stabilizers water and at least one polyalcohol, preferably mannitol or sorbitol or mixtures thereof.

The term "spasticity" as used herein refers to a muscle with spastic activity. Spasticity is defined as a motor disorder characterised by a velocity-dependent increase in tonic stretch reflexes (muscle tone) with exaggerated tendon jerks, resulting from hyperexcitability of the stretch reflex as one component of the upper motoneuron syndrome (3) Spasticity may also include focal muscle hypertonia with dystonic features (Simpson D.M, Alexander N.D, O'Brien C.F, Tagliati, M., Aswad A. S, Leon J.M, Gibson J, Mordaunt J.M., Monaghan E.P, "Botulinum toxin type A in the treatment of upper extremity spasticity"; Neurology 46, 1306-1310, May 1996).

The term "cerebrovascular event" or "cerebrovascular accident" can be manifold and refers to, for example, ischemic as well as hemorrhagic events. (Principles of Neurology, R. D. Adams/M. Victor eds., McGraw-Hill).

The term "spinal cord injury" is a traumatic lesion of neural elements in the spinal cord, resulting in any degree of sensory and/or motor deficit, autonomic dysfunction, and bladder/bowel dysfunction. The neurologic deficit or dysfunction can be temporary or permanent and may be incomplete or complete. The exact effects of a spinal cord injury may vary according to the type and level injury, and can be organized into two types:
- *Complete injury:* In a *complete injury,* there is no voluntary function below the level of the injury. Voluntary movement is impossible and physical sensation is impossible. Complete injuries are always bilateral, that is, both sides of the body are affected equally.
- *Incomplete injury:* A person with an *incomplete injury* retains some sensation below the level of the injury. Incomplete injuries are variable, and a person with such an injury may be able to move one limb more than another, may be able to feel parts of the body that cannot be moved, or may have more functioning on one side of the body than the other.

In addition to a loss of sensation and motor function below the point of injury, individuals with spinal cord injuries will often experience other changes. A major problem occurring after spinal cord injury is spasticity.

The term "onset of treatment is less than X days after .." refers to the period between initial observation of the first disease symptom and the beginning of the treatment with botulinum toxin. According to the teaching of the present invention, treatment of a spastic muscle with botulinum toxin is triggered by the observation of spasticity in a muscle as a result of a cerebrovascular event or spinal cord injury. Preferably, onset of treatment is less than 11 days, i.e. 0, 1, 2, 3, 4, 5, 6, 7, 8, 9 or 10 days after the first increased muscle activity has been observed.

In a more preferred embodiment of the present invention, onset of treatment is less than 5 days after the cerebrovascular event or spinal cord injury or after the first increased muscle activity has been observed. The term "less than 5 days" means 0, 1, 2, 3 or 4 days.

In a more preferred embodiment of the present invention, onset of treatment is less than 3 days after the cerebrovascular event or spinal cord injury or after the first increased muscle activity has been observed. The term "less than 3 days" means 0, 1 or 2 days.

In a preferred embodiment of the present invention, onset of treatment starts upon or after manifestation of the first symptoms of spasticity. First symptoms of spasticity can develop within 1 or 2 days after the cerebrovascular event or spinal cord injury or even on the same day and may affect, for example, an upper and/or lower limb and may also affect the shoulder. The muscles that are most commonly treated for spasticity are in the upper limb Musculi pectoralis complex, latissimus dorsi, teres major, brachioradialis, biceps, brachialis, pronator quadratus, pronator teres, flexor carpi radialis, flexor carpi ulnaris, flexor digitorum superficialis, flexor digitorum profundus, flexor pollicis brevis and opponens pollicis. In the lower limb, the most commonly treated muscles are medial hamstrings, lateral hamstrings, gastrocnemius medial, gastrocnemius lateral, soleus, tibialis posterior, flexor digitorum longus, flexor digitorum brevis, flexor hallicus longus, and extensor hallicus longus.

In preferred embodiment of the present invention, cerebrovascular event is can involve stroke, multiple sclerosis, spinal cord injury, head injury, cerebral palsy, hereditary spastic disease and amyotrophic lateral sclerosis or an upper motor neuron lesion.

### All terms are defined hereafter:

The term "cerebral stroke" or "stroke" are used synonymously and refer to a disease that affects the blood vessels that supply blood to the brain. A stroke occurs when a blood vessel that brings oxygen and nutrients to the brain bursts or is blocked by a blood clot or some other mass. The blood vessel may be affected not only by *internal* but also *external* processes such as tumour growth. Because of this rupture or blockage, part of the brain does not get the blood and oxygen it needs. Deprived of oxygen, nerve cells in the affected area of the brain cannot work and die within minutes. As a result, the part of the body or the brain controlled by the affected nerve cells cannot function properly. The devastating effects of a severe stroke are often permanent.

Post-stroke spasticity is a prominent example of a spastic condition triggered by a stroke. The term "post-stroke spasticity" relates to spasticity occurring after a stroke incident. Stroke is a leading cause of long-term disability, with spasticity occurring in 19% (2) to 38% of patients (Watkins CL, Leathley MJ, Gregson JM, Moore AP, Smith TL, Sharma AK. Prevalence of spasticity post stroke. Clin Rehabil 2002; 16(5): 515-522). Spasticity is defined as a motor disorder characterized by a velocity-dependent increase in tonic stretch reflexes (muscle tone) with exaggerated tendon jerks, resulting from hyperexcitability of the stretch reflex, as one component of the upper motor neuron syndrome. In some patients spasticity can be beneficial, as in the case of hip and knee extensor spasticity, which may allow weight bearing, with the affected limb acting like a splint. However, in the majority of patients spasticity causes difficulties with activities of daily living, such as dressing and cleaning the palm of the clenched hand. In accordance with the teaching of the present invention, common clinical patterns of deformity associated with spasticity in the corresponding muscle groups are treated with botulinum toxin.

There are two main types of stroke. *Ischemic stroke* is caused by blockage of a blood vessel, whereas *Hemorrhagic stroke* is caused by bleeding. Bleeding strokes have a much higher fatality rate than strokes caused by clots, often due to the space-occupying process.

While vessel-related processes may cause ischemic or hemorrhagic lesions, spasticity might also be caused by trauma, space-occupying lesions (such as tumours) and/or malformations. Accordingly, the present invention also relates to trauma, space-occupying lesions (such as tumours) and/or malformations in each of the disclosed embodiments.

"Multiple sclerosis" is an autoimmune disorder of the central nervous system, which results in the demyelinization of nerves. The immune system attacks myelin in the brain and spinal cord or both, interfering with the nerve pathways. Whether the disease manifests in repeated episodes of inflammation or as a chronic condition, it results in multiple scars, or scleroses, on the myelin sheath, leading to impairment or loss of nerve function such as numbness, tingling, loss of control of certain bodily functions, and paralysis. Multiple sclerosis can also lead to spasticity, a condition that primarily affects the lower limb.

### There are two types of multiple sclerosis related spasticity:

In flexor spasticity, mostly involving the hamstrings, and hip flexors; the hips and knees are bent and difficult to straighten.

In extensor spasticity, involving the quadriceps and adductors, the hips and knees remain straight with the legs very close together or crossed over at the ankles.

Spasticity may be aggravated by sudden movements or position changes, muscle tightness ("adaptive shortening"), extremes of temperature, humidity, or infections, and can even be triggered by tight clothing.

"Spinal cord injury" or "spinal cord accident" or "spinal cord lesion" relate to a traumatic lesion of neural elements in the spinal cord, resulting in any degree of sensory and/or motor deficit, autonomic dysfunction, and bladder/bowel dysfunction. The neurologic deficit or dysfunction can be temporary or permanent and may be incomplete or complete and may affect both sides of the body. The common types of spinal cord injury or spinal cord lesion are: (a) trauma such as car accidents, gunshots, falls, or (b) disease: polio, spina bifida, Friedrich's ataxia or (c) vascular (e.g. ischemic infarction). A major problem after spinal cord injury is spasticity.

As used herein, the term "head injury" refers to a trauma to the head that involves injury to the brain. Common causes of head injury are traffic accidents, home and occupational accidents, falls, and assaults. Head injuries may include injuries to the brain and other parts of the head, such as the scalp and skull. If intracranial hemorrhage, or bleeding within the brain occurs, a haematoma within the skull can put pressure on the brain. Complications include focal neurologic deficits, such as paralysis and spasticity.

"Cerebral palsy" is an umbrella-like term used to describe a group of chronic disorders impairing control of movement that appear in the beginning of life and generally do not worsen over time. The disorders are caused by faulty development or damage to motor areas of the brain that disrupts the brain's ability to control movement and posture. Symptoms of cerebral palsy include difficulty with fine motor tasks (such as writing or using scissors), difficulty maintaining balance or walking and/or involuntary movements. As pointed out above, "Cerebral palsy" describes a wide spectrum of pyramidal dysfunctions causing paresis, extrapyramidal dysfunctions causing dystonia, rigidity, spasticity and spasms, apraxic components and coordinative dysfunctions. Cerebral palsy (Koman LA, Mooney JF, Smith BP, Goodman A, Mulvaney T. Management of spasticity in cerebral palsy with botulinum - A toxin: report of a preliminary, randomized, double-blind trial. J Pediatr Orthop 1994; 14(3): 299-303*. (ID 1767458);* Pidcock FS. The emerging role of therapeutic botulinum toxin in the treatment of cerebral palsy. J Pediatr 2004; 145(2 Suppl): S33-S35*. (ID* 2994781)) may occur after brain hemorrhage, asphyxia, premature birth and other perinatal complications. It is a life-long condition causing uncoordinated movements, paresis and various forms of muscle hyperactivity. Patients affected by cerebral palsy, when treated in accordance with the methods disclosed herein, experience a functional improvement of hyperactive muscles.

Cerebral palsy is classified according to the type of motor impairment (Krigger KW: Cerebral palsy: an overview. Am Fam Physician 2006; 73(1):91-100*;* Green L, Greenberg GM, & Hurwitz E: Primary care of children with cerebral palsy. Clin Fam Prac 2003; 5(2):467-491):
- Spastic
   • >70% of cases
   • Caused by damage to the pyramidal brain structures
   • May be hemiplegic, diplegic, or quadriplegic
- Dyskinetic (athetoid)
   • 10% to 20% of cases
   • Caused by inadequate regulation of muscle tone and coordination
   • Associated with severe neuromuscular dysfunction
- Ataxic
   • 5% to 10% of case
   • Usually caused by cerebellar dysfunction
   • Associated with disturbance in coordination of voluntary movements due to muscle dyssynergia
- Mixed
   • Elements of spasticity and dyskinesia
   • Usually more global damage present

In a population-based study of the prevalence of cerebral palsy (CP) over a 16-year period for 1-year survivors, spastic quadriplegia was the predominant type of CP overall in the majority of birth weight groups. The greatest increase over time was in the proportion of very low weight infants (less than 1500 g) with spastic diplegia, from 7% in 1975-1977 to 32% in 1986-1991 (Winter S, Autry A, Boyle C, et al: Trends in the prevalence of cerebral palsy in a population-based study. Pediatrics 2002; 110(6):1220-1225*).*

"Hereditary spastic paraplegia", also called familial spastic paraplesis, spastic spinal familial paralysis, Strumpell-Lorrain syndrome, Strumpell disease, or Troyer syndrome refers to a group of inherited disorders that are characterized by progressive and usually severe weakness and spasticity of the lower limbs. It affects the corticospinal tracts within the spinal cord. The disease may result in progressive spasticity of leg muscles with varying degrees of stiffness and weakness of other muscles groups in the tights, lumbar spinal area, and muscles responsible for up and down feet movements. When the only manifested symptom is progressive spasticity, hereditary spastic paraplegia is also known as Pure Hereditary Spastic Paraplegia. The extent of degeneration and severity of symptoms may vary among the affected people, even those among the same family group. The age of onset for the disease may also vary. Some families show a pattern of disease, with symptoms developing earlier in each new generation. In most individuals, however, the disease onset occurs between the second and fourth decades of life, with a few cases beginning later, as as early as infancy and early childhood.

"Amyotrophic lateral sclerosis", or Lou Gehrig disease is an adult-onset disease of progressive neurodegeneration affecting both upper and lower motor neurons (Patel SA & Maragakis NJ: Amyotrophic lateral sclerosis: pathogenesis, differential diagnoses, and potential interventions. J Spinal Cord Med 2002; 25(4):262-273.; Rowland LP & Shneider NA: Amyotrophic lateral sclerosis. N Engl J Med 2001; 344(22):1688-1700*).* The etiology is likely multifactorial, involving both genetic and environmental factors. Amyotrophic lateral sclerosis is also known as motor neuron disease (Ashworth NL, Satkunam LE, Deforge D, Treatment for spasticity in amyotrophic lateral sclerosis/motor neuron disease., Cochrane Database Syst Rev. 2006 (1): CD004156*).* Spasticity commonly affects patients with motor neuron disease and it is likely to contribute to worsening muscle dysfunction, increased difficulty with activities of daily living and deteriorating quality of life *(*Ashworth NL, Satkunam LE, Deforge D, Treatment for spasticity in amyotrophic lateral sclerosis/motor neuron disease., Cochrane Database Syst Rev. 2006 (1): CD004156) Spasticity occurs from loss of inhibition from upper motor neurons.

An "upper motor neuron lesion" is a lesion of the neural pathway above the anterior horn cell or motor nuclei of the cranial nerves. This is in contrast to a lower motor neuron lesion, which affects nerve fibers traveling from the anterior horn of the spinal cord to the relevant muscle(s).

In preferred embodiment of the present invention, the spasticity is a spasticity affecting an upper and/or lower limb, the upper limb optionally comprising the shoulder. As used herein, the term "upper limb" refers to the arm comprising the shoulder and, thus, may involve muscles such as for example (a) shoulder: pectoralis complex, latissimus dorsi, teres major and subscapularis, (b) elbow: brachioradialis, biceps, brachialis, (c) Forearm: pronator quatratus, pronator teres, (d) Wrist: flexor carpi radialis, flexor carpi ulnaris, (e) Thumb: flexor pollicis longus, adductor pollicis, flexor pollicis brevis/opponens pollicis, (f) Fist/hand: flexor digitorum superficialis, flexor digitorum profundus, lumbricales/interossei, As used herein, the term "lower limb"comprises the hip, thigh, leg, knee, ankle, foot and the toes and, thus, may involve muscles such as for example (a) Hip: iliopsoas, psoas, rectus femoris, (b) Knee: medial hamstrings, gastrocnemius, lateral hamstring, quadriceps mechanism, (c) Thighs: adductor longus, adductor brevis, adductor magnus, (d) Foot: gastrocnemius medial, gastrocnemius lateral, soleus, tibialis posterior, tibialis anterior, flexor digitorum longus, flexor digitorum brevis, flexor hallucis longus, (e) Toes: extensor hallucis longus.

The present invention also relates to a method of treatment of spasticity related to a cerebrovascular event or spinal cord injury, the method comprising administering to a patient a composition comprising an effective amount of a Clostridium botulinum toxin, wherein
(a) the composition comprises:
   i. the neurotoxic component of the Clostridium botulinum toxin complex, but is free of the complexing proteins of the botulinum toxin complex, or
   ii. the neurotoxic component and, in addition, the complexing proteins of the botulinum toxin complex; and
(b) the composition is administered less than 11 days after the cerebrovascular event; and
(c) the composition is administered by injection.

According to the joints that are affected by spasticity, the neurotoxic component is usually injected as follows :

**Table 1 Table Management of Spasticity of the upper limbs with Botulinum Toxin Type A (adapted from "WE MOVE")**

| **Clinical Pattern** | **Potential Muscles Involved** | **BTXCo Dose Units/Visit** | **Number of Injection Sites** |
|---|---|---|---|
| **Upper limb:** | | | |
| Adducted/Internally | *pectoralis complex* | 75-150 | 2-4 |
| Rotated Shoulder | *latissimus dorsi* | 50-150 | 3-4 |
| | *teres major* | 25-100 | 1-2 |
| | *subscapularis* | 50-100 | 1-2 |
| Flexed Elbow | *brachioradialis* | 25-100 | 2 |
| | *biceps* | 75-200 | 2 |
| | *brachialis* | 40-100 | 2 |
| Pronated Forearm | *pronator quadratus* | 10-50 | 1 |
| | *pronator teres* | 25-75 | 1 |
| Flexed Wrist | *flexor carpi radialis* | 25-100 | 2 |
| | *flexor carpi ulnaris* | 20-70 | 2 |
| Thumb-in-Palm | *flexor pollicis longus* | 10-30 | 1 |
| | *adductor pollicis* | 5-25 | 1 |
| | *flexor pollicis brevis*/*opponens* | 5-25 | 1 |
| | *pollicis* | | |
| Clenched Fist | *flexor digitorum superficialis* | 20-40 | 1 |
| | *(per fascicle)* | | |
| | *flexor digitorum profundus* | 20-40 | 1 |
| | *(per fascicle)* | | |
| Intrinsic Plus Hand | *lumbricales*/*interossei* | 5-10 | 1 |
| | *(per lumbrical)* | | |

| **Lower limb:** | | | |
|---|---|---|---|
| Flexed hip | *iliopsoas* | 50-200 | 2 |
| | *psoas* | 50-200 | 2 |
| | *rectus femoris* | 75-200 | 3 |

The following examples are provided by means of illustration only, and are not intended to be limiting.

### Example 1: Botulinum Toxin Therapy for Treatment of Spasticity 5 days after a stroke.

A 53 year-old female patient suffering from a stroke and a resulting spasticity in the muscles of the right extremities within the first week after stroke, was evaluated for botulinum toxin therapy. The CCT demonstrated a severe demarcation of the infarction area (MCA) of the left hemisphere. After all appropriate examinations an injection scheme was constructed and botulinum toxin is applied accordingly in a total dose of 200MU (elbow flexors and flexors of the forearm). On re-evaluation after 6 weeks the symptomatology was markedly improved. After 3 months reinjection was necessary due to the increasing tone. The CCT showed a much improved situation, and voluntary movements were possible. The patient is still under treatment with botulinum toxin injections.

The patient is currently presenting with only minor spasticity that is considerably less than one would have expected from CT-findings. She is just slightly handicapped and not depending on assistance.

### Example 2: Botulinum Toxin Therapy for Treatment of Adductor Spasticity

A 24- year-old patient presents with adductor spasticity that has been increasing for three 3 days. Urinary retention is another recent complaint. The patient is known to have MS with intermittent episodes. MRI of the thoracic spine was indicative of focal lesion at T11 with uptake of contrast medium. The patient was informed about therapeutic options and decided in favor of Botulinum toxin injections. One vial, i.e. 100 MU (total dose 200 MU) was injected per side. At the same time, she had treatment of her acute focal inflammation by massive-dose cortisone. An effect on the adductors was appreciable after six days, meaning that there was but mildly increased muscle tone now, and the patient remained mobile throughout the course. Urinary retention was improved within four days without any drug application, so the catheter could be removed again. The adductor muscles were reinjected after 12 weeks. MRI follow-up four months later confirmed nice regression of the inflammatory focus. Additional BTX-injections were not necessary.

We know that intermittent MS is associated with typical symptoms, but we don't know to what extent they may be remitting, persisting or aggravating. An efficient treatment with complete remission is thus making a lot of sense apart from being also called for. In the above case, we were not only able to preserve the patient's mobility but also to stimulate bladder evacuation (triggering of bladder spasticity by adductor spasticity). Systemic and/or permanent effects were avoided.

### Example 3: Botulinum Toxin Therapy for Treatment of Incomplete High Paraplegia

A 19-year-old young man is presented, who suffered incomplete high paraplegia (C4) in a bathing accident - diving into a swimming pond. His condition was marked by atonic paralysis distally of T11 during the first seven weeks, subsequently he developed tetraspasticity predominantly on the left, with hip flexion and autonomous bladder function. Due to this hip flexion, the patient was unable to sit in a wheelchair since touch would cause involuntary violent flexion on the left. He was injected 150 MU into his left hip flexor. This resulted in sufficient paresis, so he could sit in the wheelchair, and his mother was able to manage his transfer to the supine position on her own. After three injections, his symptoms were substantially improved to the degree that relevant flexor spasticity was no longer observed. The patient is undergoing treatment still for detrusor hyperactivity - with Botulinum toxin (200 MU) injected in the detrusor muscle.

## Claims

1. *Clostridium botulinum* neurotoxin for the treatment of spasticity related to a cerebrovascular event or to a cerebral or spinal cord injury, wherein onset of treatment is less than 11 days after the cerebrovascular event or cerebral or spinal cord injury or the first onset of spasticity.

2. The neurotoxin of claim 1, wherein onset of treatment is less than 5 days after the cerebrovascular event or cerebral or spinal cord injury.

3. The neurotoxin of claim 2, wherein onset of treatment is less than 3 days after the cerebrovascular event or cerebral or spinal cord injury.

4. The neurotoxin of any one of claims 1 to 3, wherein onset of treatment starts upon or after manifestation of the first symptoms of spasticity.

5. The neurotoxin of any one of claims 1 to 4, wherein the cerebrovascular event is related to or involves stroke, multiple sclerosis, spinal cord injury, head injury, cerebral palsy, hereditary spastic disease and amyotrophic lateral sclerosis or an upper motor neuron lesion.

6. The neurotoxin of any one of claim 1 to 5, wherein the spasticity is a spasticity affecting an upper and/or lower limb, the upper limb optionally comprising the shoulder.

7. The neurotoxin of any one of claims 1 to 6, wherein the *Clostridium botulinum* is selected from the group consisting of serotype A, B, C, D, E, F or G including subtypes or mixtures thereof.

8. The neurotoxin of claim 7, wherein the *Clostridium botulinum* is of serotype A.

9. The neurotoxin of any one of claims 1 to 8, being free of any complexing proteins of the *Clostridium botulinum* toxin complex.

10. The neurotoxin of any one of claims 1 to 8, being in the form of a *Clostridium botulinum* toxin complex.

11. The neurotoxin of any one of claims 1 to 10, which comprises a mammalian serum albumin.

12. A method of treatment of spasticity related to a cerebrovascular event, the method comprising administering to a patient a composition comprising an effective amount of a *Clostridium botulinum* neurotoxin, wherein
(a) the composition comprises:
(i) the neurotoxic component of the *Clostridium botulinum* toxin complex, being free of the complexing proteins of the botulinum toxin complex, or
(ii) the botulinum toxin complex; or mixtures thereof and
(b) the composition is administered less than 11 days after the cerebrovascular event; and
(c) the composition is administered by injection.

13. The method of claim 12, wherein a composition as defined in any of claims 2 to 11 is used.
